# EUROPEAN PATENT SPECIFICATION

(11) **EP 1 727 553 B1**
(45) Date of publication and mention of the grant of the patent: **09.07.2008**
(21) Application number: 04820384.8
(22) Date of filing: 16.12.2004
(51) Int. Cl.: A61K 36/185, A61P 7/08

(54) **AGENT FOR SUBSTITUTION OF BLOOD PLASMA AND A METHOD OF ITS PRODUCTION**
MITTEL ZUR SUBSTITUTION VON BLUTPLASMA UND HERSTELLUNGSVERFAHREN
AGENT DE SUBSTITUTION DU PLASMA SANGUIN ET SON PROCEDE DE PRODUCTION

(30) Priority: 16.12.2003 CZ 20033424
(43) Date of publication of application: 06.12.2006
(73) Proprietor: Parenteral, A.S., 500 11 Hradec Králové (CZ)
(72) Inventor: HORSKA, Irena, 412 00 Liberec (CZ)
(74) Representative: Fischer, Michael
(86) International application number: PCT/CZ2004/000087
(87) International publication number: WO 2005/058332

(56) References cited:
- WO-A-03/018639
- GB-A- 886 487

## Description

### Field of Technology

The invention relates to an agent for substitution of blood plasma and a method of its production.

### Background of the Invention

Blood plasma is a transparent liquid blood component of a yellowish colour, that contains a wide spectrum of proteins, sugars, fats, and minerals in addition to water. It is playing a major role in nutrient conveyance, metabolic processes, and information transmission. The blood plasma represents some 55 % of blood in volume. Absolute majority of the plasma component is being restored in the cycle of several hours down to several days. Its comprehensive structure and the roles played by its individual components still remain unknown in exact terms. For the transfusion purposes, the blood plasma, once taken from a donor, or during the taking itself already, gets separated very quickly from other blood components by a so-called plasmophoresis, and is then frozen down swiftly. Shelf life of such plasma can be up to 2 years when frozen down and kept at about -25 °C. The reasons behind the blood plasma administration might include a substantial loss of proteins from the human body, like upon burns, large-scale blood losses, as one of role played by the proteins is to maintain the blood osmolarity. It is often possible to administer instead of the blood plasma a preparation from the group of infusion solutions, such as a physiological solution, Hartman solution, Ringer-lactate solution with the similar or simpler ionic composition as the blood plasma; furthermore, the colloid, so-called plasmatic expanders, based on the glycidic substances, such as dextran, hydroxyethylene starch or and albumin which, however in respect of their composition cannot become any full-valued blood plasma substitute. The plasma substitutes used until now (widely produced dextran, gelatins, hydroxyl ethyl-starches) are the macromolecular medicaments having nothing in common with plasma, which are being quickly bio-transformed in the liver, and can only be used to maintain the blood volume where there is a life threat and where the organism shock treatment is commenced because of the shock from e.g. burns or sepsis, but they should not be used to maintain the plasmatic volume upon a loss of proteins, water, and electrolytes, when in such events one has to apply directly the blood plasma or the plasmatic protein fractions with large quantities of albumins. Albumin is one of the plasmatic proteins, most abundant in the plasma, but unable to stimulate the organism's immunity system. The immunity system supporting globulin is about thrice less contained than globulin in plasma, and this is why the blood plasma is not produced from globulin. The blood plasma is composed of about 91 % water, 7 % dissolved organic substances such as albumins, globulins, fibrinogen, 1 % dissolved inorganic substances with the ions of Na⁺ (150 mmol/l), Cl⁻ (100 mmol/l), HCO₃ (30 mmol/l), K⁺ (5 mmol/l) and rest of hormones and other substances being conveyed. There are two main reasons why a practical need to substitute the human blood plasma used until now by other preparations that would feature similar functional characteristics is being ever more frequently considered. Currently, the number of the homolog blood transfusions, i.e. the transfusions of the blood from anybody else, but having the same or similar antigenic properties, to the surgical or other patients is dropping. Primary cause behind this decline in the blood transfusions is the price for the healthy blood and blood derivates; in the U.S., for example, the costs have climbed up to 500 dollars per transfusion. Additional increases in the transfusion medicine costs can be expected because of scarcity of healthy donors and the necessity to cure ever more frequently the complications caused by the transfusion itself or by the blood plasma application. Another reason buttressing the development of the laboratory, preclinical, and clinical research carried out in the strive for substitution of one or more functions of the blood rests in the complications that tend to follow the transfusions and blood plasma applications. Among the most widespread ailments ascribable to blood transfusions, there is chronic hepatitis, liver cirrhosis, weakened resistance to tumors, and other infections, mainly HIV, BSE, SARS, LYME, borreliosis, retroviruses, prions, and others. As estimated by the American disease monitoring centers, 3.500 people died yearly on the hepatitis communicated via a blood or plasmatic transfusion in the last 70's, while other figures reach several times higher. Thanks to a more profound testing and careful selection of donors the number of contamination events ascribable to the Hepatitis B virus has fallen down, but a new form of Hepatitis C virus appeared, having attacked more than 4 million Americans, and several hundred thousand people had contracted the disease upon blood transfusion or blood plasma administration. Additional rigorous testing made it possible to reduce the number of the post-transfusion Hepatitis C occurrences. Hepatitis G is a recently described RNA virus, being demonstrably contagious via transfusions, even though the infection risks are not normally known. There are therefore the concerns that a new infectious hazard might appear, recognizable only when a handful of the blood recipients take infection. A cytomegalovirus-evoked infection (CMV) need not manifest itself outwardly in case of most blood recipients, but can be serious up to infaustic with the immunosuppressed patients without any CMV antidotes (newly born infants with low birth weight, transplanted patients). A particular attention is being paid to the blood/plasma contamination with the HIV virus, as both the children and adults caught the infection in numerous countries (in France e.g., there are six to eight thousand people who contracted HIV after transfusions and blood plasma administration in the period from 1982 to 1985). In Africa, there is also an increase in HIV, ascribable not only to sexual promiscuity, but some 10 % of the infections resulted from blood transfusions and blood plasma. The percentage is even higher in Pakistan, now close to one half of all AIDS occurrences. Undoubtedly, it is closely related to inadequate coordination of the donors' blood testing in the developing countries. The above findings pose now a serious problem worldwide.

Objective of the invention is to provide for a valuable blood plasma substitute of the protein type, featuring the similar physical and chemical characteristics as the human globulin, capable to replace it, having an adequate osmotic response and viscosity, being inert to viruses and supporting the bodily immunity, capable of biotransformation to immunoglobulins.

Objective of the invention is moreover to provide for a suitable initial product for the blood plasma substitute and a reliable and efficient method of its production.

### Subject-matter of the invention

To a major extent, the disadvantages of the existing state of art are being annulled and the objective of the invention achieved by the agent for substitution of the blood plasma, formed by the vegetal protein substance edestin, which is advantageously being produced from the hemp seeds. Advantageously, edestin is a component of the solution comprised of 5 % - 40 % in weight of edestin, 0.5 % - 7 % in weight of inorganic substances, 0.5 % - 7 % in weight of organic substances, hormones, and other substances to 100 % in weight of the colloid solution. According to the method of production of the agent for blood plasma substitution, the vegetal seeds are either cold-pressed or ground to powder and extracted under a compressed gas; the pressed out or extracted oil is separated, the pressed together or extracted seeds to be relieved of fat and decolorized are extracted by action of the organic solvents, the fat relieved and decolorized substance is then filtered out and exposed to action of the inorganic salts in the water solution and at the temperature 20 °C - 60 °C, the solution is filtered and edestin is finally isolated from the filtrate. Advantageously, the hemp seeds are subjected to the cold pressing and grinding processes and to the gas-assisted pressure extraction. The vegetal seeds are being extracted under the compressed carbon dioxide. Advantageously, a saturate solution of the inorganic salts at the temperature 20 °C - 60 °C (20 °C advantageously) is added to the fat relieved and decolorized substance; the resulting mixture is then being stirred for 0.01 - 2.5 hour (0.5 hour advantageously) at the temperature from 20 °C to 60 °C (20 °C advantageously); then, 0.1% - 30% in weigh of the inorganic salt is added; the resulting mixture is then being stirred for 0.6 - 60 minutes (30 minutes advantageously) at the temperature 20 °C - 60 °C; filtrated by means of chemical filters; the filtrate is sharply cooled down to -50 °C - +15 °C in order that edestin would crystallize; edestin raw crystals are poured into the distilled water and separated by pressure filtration; the resulting filtrate is used to make a water solution with edestin or the fat relieved and decolorized substance, once water is added, is used to make the water solution at the temperature 20 °C - 60 °C (5 °C advantageously); an inorganic salt, such as sodium chloride advantageously, is added to the water solution to give 1% - 20% solution (5% solution advantageously); the mixture is then being stirred intensely for 1 - 60 minutes (30 minutes advantageously); then it is left to settle down and sediment for the protein sediment separation; the protein sediment is filtered; in the filtrate, edestin is isolated in the form of a solution by action of a salt (ammonium sulfate advantageously) and in both cases the edestin is further purified up to the purity level higher than 95 % (99.9 % advantageously) by means of dialysis or column chromatography.

In its very nature, edestin is a globulin (medium-sized protein) of the vegetal origin, consisting of a specific primary sequence of amino acids, being in its composition and amino-acid-based structure very similar, close to the natural human globulins. Globulin is a protein, which together with the albumins makes the largest portion of the blood plasma's simple proteins. Most antibodies (immunoglobulin types) are contained in the human blood plasma's globulin fraction. Edestin is comprised of amino acids (with C, O, H as their basic building blocks). As one of the proteins, edestin according to the invention replaces globulin as the protein of the human origin. Vegetal edestin seems to be a convenient precursor (predecessor) of the immunoglobulin synthesis (is arranging for this synthesis; thus, the organism will get a semi-finished human specific immunoglobulins and does not need to make them itself in the complicated and energy-consuming processes). The human blood plasma comprises three types of the plasmatic proteins: globulin series, albumin series, and one type of fibrinogen. Edestin can be used in place of globulin. Plasmatic protein mostly originates in the liver, the human body is itself producing the globulins in the liver and immunoglobulins (antibodies) in the lymphocytes, white blood cells). According to the invention, the solution provides the organism with a natural substance of the vegetal origin, closest relative to globulin-based substance, in order that the organism (mainly when suffering a disease or being weakened) does not need to produce globulin itself, saving thus its resources. In its physical and chemical characteristics edestin is like the human globulin, thus making it possible to replace the human globulin (one protein is substituted by another). It can be used and an initial product for the physical replacement of human plasma, as it has the same necessary viscosity and osmotic response, being moreover inert in contact with viruses that affect the human body, not being attached e.g. by HIV. When being split, the edestin transforms itself into the form of the fully valued human immunoglobulins, supporting thus the immunity. The osmotic and viscose parameters of an edestin solution are optimal for the construction of the plasmatic expanders (imitations). Because of its molecular weight, the edestin is not forthwith removed by the glomerular filtration in the kidneys (unlike the other contemporary substitutes - dextran, glucose, and other parent solutions), but bio-transformed gradually to immunoglobulins by action of the bodily metabolic processes taking place mainly in the liver. The above biotransformation, such as the one down to the form of the specific immunoglobulins is beneficial in respect of the edestin's immunopotentiating effects. When in a proper concentration, the edestin is meeting the limits of the colloid-osmotic blood plasma pressure. In the form according to the invention, edestin can be stored in the following conditions: dry, dark, and cold place (at the temperature around +5°C) together with the agents that absorb the climatic or atmospheric condensation humidity of the surrounding environment. As the blood plasma imitation, the invention is valuable, its component - according to the invention - has the similar physical and chemical properties as the human globulin, having the capacity to replace it, featuring the necessary osmotic response and viscosity, being inert to viruses, supporting the bodily immunity, and being able of biotransformation down to immunoglobulins. The initial product for the blood plasma substitute is widely available, can be prepared and stored easily and reliably on a long-term basis without the necessity to freeze it down.

### Example of the execution

The agent for substitution of blood plasma according to the invention implies total elimination of the above complications and risks related to the administration of a contaminated blood plasma, based on the newly conceived and suitable plasma substitute and relying on the existing knowledge of the structure and characteristics of the proteins contained in the human blood plasma. In the world of flora, there is a protein unique in its composition and nearly identical to the human protein called globulin, i.e. edestin. The plasmatic proteins of three types are present in the human plasma: globulin series, albumin series, and fibrinogen. According to the invention, the vegetal protein of edestin can replace the human globulin in order to free the human body from the duty to produce it by its own means as otherwise the body would exhaust its energy in doing so, especially when suffering a disease or physiological weakness. The plasmatic proteins are being mostly created in the liver and the immunoglobulins (antibodies) in the lymphocytes (white blood cells). Edestin is a hexametric protein of the globular type, having 300,000 D (Dalton) in molecular weight, composed of six identical protein subunits, highly stable (at pH 5.8 to 7.8, and nearly identical to an analogous globulin contained in the human plasma. In its primary structure, edestin is comprised of a variety of valuable amino acids, mainly of arginin and tryptogam. In comparison with the human blood plasma, it contains 80 % more of these valuable substances. Once dissolved in the NaCl physiological solution or in water for injections and intravenous application, edestin hydrochloride has a minimum down to zero antigenic response in the case of the experimental mammals. It is obvious from the pilot experimental knowledge that edestin does not evoke any adverse immunological responses, but that it is - on the contrary - strengthening the immunological responses in their efficiency, and this can be explained by its quick usability for the synthesis of antibodies, in particular of the proteins of the gamma-globulin series. In case of the cellular immunity it can be taken as a basis that the edestin metabolism products that resemble gamma-globulin in their structure are being bound on the surface receptors of the respective lymphocyte populations, and this is why it seems obvious to use the edestin or its fragments as an immunopotentiating preparation. In respect of its molecular weight, edestin is not expelled from the body by any direct glomerular filtration in the kidneys, being metabolized (degraded via molecular splitting) very well in the liver tissues down to the essential amino acids. A stable edestin solution will basically eliminate all the widely known disadvantages of the blood plasma application (risk of anaphylactic shock, attack by the human HIV viruses, hepatitis, BSE, SARS, Lyme borreliosis, retroviruses, prions, etc.) and of the presently widely used blood plasma substitutes based on dextrans, gelatin polymers or starch derivates, being subject to quick excretion via the kidneys. The substitutes used until now (widely produced dextrans, gelatin, hydroxyethyl starches) are macromolecular medicaments that have nothing in common with plasma, being - in contrast to edestin - readily biotransformed and can only be applied within a strive to maintain the blood volume in the life-dangerous situations or within the commencement of bodily shock treatment, after a patient has suffered e.g. burns or sepses. It is not good to use them in the strive to maintain the blood volume upon the loss of proteins, water, and electrolytes; in these events one has to use directly the blood plasma or the plasmatic protein fractions with a large number of albumins, but with all the application-related risks. Subject of the invention supercedes a lower-quality protein with much better one (of the globulin type), doing so without these risks pending, as the edestin as a vegetal globulin is inert in contact with all the known viruses being communicated through the blood plasma. In accordance with our existing studies the enriched edestin-base substitutes will outclass the blood plasma in the overwhelming majority of the clinical needs. An ideal source of edestin is available in the seeds of the hemp called *Cannabis sativa* that contain edestin in the largest amounts. Raw edestin can be extracted from these seeds and refined to high level of purity in its crystallizing and suspension forms. The edestin-based blood plasma substitute can be prepared in accordance with the process used to gain the initial product for the blood plasma substitute being subject of the invention at the purity level of up to 99.9 %, where it features nearly unlimited shelf life. The subject of invention eliminates the drawbacks of the blood plasma from the donors (complete with all the infection communication risks such as HIV, hepatitis, BSE, SARS, Lyme borreliosis, retroviruses, prions, etc.) that consists mainly of albumins and minerals and substitutes it with a substantially better preparation of the protein type and vegetal origin. Due to its globulin nature the edestin-based preparation (in contrast to the human blood plasma composed mainly of the low-molecular albumins that only maintain the osmotic blood pressure without any abilities to sustain the immunity system), is not only buttressing the blood osmotic response, but the edestin-based globulin, when being split, is moreover stimulating the bodily immunity system. The low or zero antigenic action of edestin and high content of the essential amino acids appeared to predestine the edestin as a base from which the human blood plasma substitute can be made.

In order to make up a fully valued blood plasma substitute, one needs 5% - 40 % in weight (7.2 % in weight advantageously) of edestin, 0.5% - 7 % in weight of inorganic substances, 0.5% - 7 % in weight of organic substances, hormones, and other substances complementing the colloid solution to 100 %, while their contents have to be balanced so that the osmotic-colloid values of such solution match the equivalent values of the blood plasma. As an example, a colloid solution contains 7.2 % in weight of edestin, 4 % in weight of inorganic substances, 4 % in weight of organic stuff to 100 % in weight of the colloid solution. Another example is a colloid solution with 40 % in weight of edestin, 7 % in weight of inorganic substances, and 7 % in weight of organic stuff and hormones as the balance to 100 % in weight of the colloid solution. Any concentration can be created, as necessary.

According to the method of production, the initial product for the substitution of the human blood plasma in accordance with the invention, the hemp seeds are either crushed, ground by means of the widely available technological equipment during their cold pressing already or crushed and extracted by means of a supercritical fluid extraction in the inert atmosphere of the liquid CO₂ via the CO₂ extraction. Preparation of the human blood plasma substitute from the crystalline form of edestin is being accomplished by pressing out or extraction of the hemp oil based on the CO₂ method, while the wastes in the form of ground flour are relieved of the residual oil and chlorophyll natural dye in the way that the relief of fat and decolorizing are carried out via the extraction by action of some organic solvents such as ether, chloroform, etc. Given the method of production based on the edestin coagulation in its crystalline form, one has to prepare the edestin suspension by stirring intensely the edestin crystals in the 10% to 30% physiological solution. The concentrated hydrochloric acid is being added to this suspension while it is being stirred intensely, until the solution gets fully exhausted (becomes a white solution). Lyophilization (vacuum evaporation or freezing out at -80 °C) of the solution will result in a loose substance of edestin hydrochloride in its soluble form which, when dissolved once again in the injection water or in the physiological solution will give a clear solution, useable for the intravenous application. Shelf life of the edestin loose substance reaches up to several years with its stable state preserved. Given the method of edestin production by isolation from the filtrate by action of the salts, it is desirable to use, while the water or water solution is being added to the fat relieved and decolorized substance, a lower temperature of the solution in order to suppress the activity of proteases that could split the edestin. The protein sediment filtrate is colored white and gray. The filter grade has to be selected with respect to the polysaccharide aggregates, i.e. starch that must not transfer into the filtrate. Where the edestin is to be used in practice in its suspense form as an agent for substitution of blood plasma, one has to prepare the edestin loose substance in the hydrochloride form by adding a concentrated hydrochloric acid to the edestin suspension while it is being stirred intensely, until the solution gets fully clarified (clear solution). Lyophilization (vacuum evaporation and/or freezing out at -80 °C) of the solution will result in a loose substance of edestin hydrochloride in its soluble form which, when dissolved once again in the injection water or in the physiological solution, will give a clear solution, useable for the intravenous application. Shelf life of the edestin loose substance reaches up to several years with its stable state preserved.

## Claims

1. Agent for substitution of the blood plasma,
**characterised by that**
it is formed by the vegetal protein substance edestin.

2. Agent for substitution of the blood plasma according to the claim 1,
**characterised by that**
it is a product of hemp seeds.

3. Agent for substitution of the blood plasma according to the claim 1 or 2,
**characterised by that**
it is a component of a solution comprising 5 % - 40 % in weight of edestin, 0.5 % - 7 % in weight of inorganic substances, 0.5 % - 7 % in weight of organic substances, hormones and other substances to 100 % in weight of the colloid solution.

4. Method of production of the agent for blood plasma substitution according to the claim 1,
**characterised by that**
the vegetal seeds are either cold-pressed or ground to powder and extracted under a compressed gas; the pressed out or extracted oil is separated, the pressed together or extracted seeds are extracted by action of the organic solvents for relief of fat and color, the fat relieved and decolorized substance is then filtered out and exposed to action of the inorganic salts in the water solution and at the temperature 20 °C - 60 °C, the solution is filtered and edestin is finally isolated from the filtrate.

5. Method of production of the agent for blood plasma substitution according to the claim 4,
**characterised by that**
the hemp seeds are subjected to the cold pressing and grinding processes and to a gas-assisted pressure extraction.

6. Method of production of the agent for blood plasma substitution according to the claim 4 or 5,
**characterised by that**
the vegetal seeds are being extracted under the compressed carbon dioxide.

7. Method of production of the agent for blood plasma substitution according to one of the claims 4 - 6,
**characterised by that**
- a saturate solution of the inorganic salts at the temperature 20 °C - 60 °C, 20 °C advantageously is added to the fat relieved and decolorized substance;
- the resulting mixture is then being stirred for 0.01 - 2.5 hour (0.5 hour advantageously) at the temperature 20 °C - 60 °C, 20 °C advantageously;
- 0.1% - 30% in weigh of inorganic salt is added;
- the resulting mixture is then being stirred for 0.6 - 60 minutes, 30 minutes advantageously, at the temperature 20 °C - 60 °C;
- filtrated by means of chemical filters;
- the filtrate is sharply cooled down to -50 °C - +15 °C in order that edestin would coagulate in a crystal form;
- edestin raw crystals are poured into the distilled water and separated by pressure filtration;
- the resulting filtrate is used to make a water solution with edestin or the fat relieved and decolorized substance,
or
- by adding water to the fat relieved and decolorized substance, a water solution of the temperature 20 °C - 60 °C, 5 °C advantageously, is formed;
- an inorganic salt, such as sodium chloride advantageously, is added to the water solution to give 1% - 20% solution, 5% solution advantageously;
- the mixture is being stirred intensively for 1 - 60 minutes, 30 minutes advantageously;
- it is left to settle down and sediment for the protein sediment separation;
- the protein sediment is filtered;
- in the filtrate, edestin in the form of a solution is isolated by action of a salt, ammonium sulfate advantageously
and in both cases the edestin is further purified by means of dialysis or column chromatography up to the purity level higher than 95 % (99.9 % advantageously).

## Patentansprüche

1. Mittel für den Ersatz von Blutplasma,
**dadurch gekennzeichnet, dass**
es durch den Pflanzeneiweißstoff Edestin gebildet wird.

2. Mittel für den Ersatz von Blutplasma gemäß Anspruch 1,
**dadurch gekennzeichnet, dass**
es ein Produkt aus Hanfsamen ist.

3. Mittel für den Ersatz von Blutplasma gemäß Anspruch 1 oder 2,
**dadurch gekennzeichnet, dass**
es eine Komponente einer Lösung ist, die 5 - 40 Gewichtsprozente von Edestin, 0,5 - 7 Gewichtsprozente von anorganischen Stoffen enthält.

4. Herstellungsart des Mittels für den Ersatz von Blutplasma gemäß Anspruch 1,
**dadurch gekennzeichnet, dass**
die Pflanzensamen entweder kaltgepresst oder gemahlen oder unter Gasdruck extrahiert werden, das ausgepresste oder extrahierte Öl abgetrennt wird, die gepressten oder extrahierten Samen nach Entfettung und Entfärbung durch die Einwirkung von anorganischen Lösungsmitteln extrahiert werden, die entfettete und entfärbte Masse weggefiltert wird, sie bei Temperaturen von 20 bis 60 °C in einer Wasserlösung der Einwirkung von anorganischen Salzen ausgesetzt wird, die Lösung gefiltert und aus dem Filtrat das Edestin isoliert wird.

5. Art der Herstellung des Mittels für den Ersatz von Blutplasma gemäß Anspruch 4,
**dadurch gekennzeichnet, dass**
die Hanfsamen dem Prozess des Kaltpressens und Zermalens und dem Prozess der Druckextraktion unterzogen werden

6. Art der Herstellung des Mittels für den Ersatz von Blutplasma gemäß Anspruch 4 oder 5,
**dadurch gekennzeichnet, dass**
die Pflanzensamen unter dem Druck von Kohlendioxid extrahiert werden.

7. Art der Herstellung des Mittels für den Ersatz von Blutplasma gemäß einem von Ansprüchen 4 bis 6,
**dadurch gekennzeichnet, dass**
- der entfetteten und entfärbten Masse gesättigte Lösung von anorganischen Salzen mit Temperatur 20 bis 60 °C, mit Vorteil mit Temperatur 20 °C, zugefügt wird
- die entstandene Mischung bei Temperatur 20 bis 60 °C, mit Vorteil 20 °C, während 0,01 bis 2,5 Stunden, mit Vorteil 0,5 Stunden gemischt wird,
- danach 0,1 - 30 Gewichtsprozente anorganischer Salze zugefügt werden,
- die entstandene Mischung bei Temperatur 20 bis 60 °C während 0,6 - 60 min., mit Vorteil während 30 min. gemischt wird,
- mit Hilfe von chemischen Filtern gefiltert wird,
- das Filtrat rasch auf die Temperatur - 50 bis + 15 °C wegen der Ausflockung des Edestins in kristallinischer Form abgekühlt wird,
- die Edestinrohkristalle in destilliertes Wasser geschüttet und mit Druckfiltration separiert werden,
- aus dem entstandenen Filtrat eine Wasserlösung mit Edestin gebildet wird
oder
- aus der entfetteten und entfärbten Masse durch die Zugabe von Wasser eine Wasserlösung mit Temperatur 20 - 60 °C, mit Vorteil 5 °C gebildet wird,
- in die Wasserlösung anorganisches Salz, mit Vorteil Natriumchlorid so zugefügt wird, dass eine 1 - 20 % Lösung, mit Vorteil 5 % Lösung entsteht,
- die Mischung intensiv 1 bis 60 min., mit Vorteil 30 min. gemischt wird,
- man sie wegen der Abtrennung des Eiweißsedimentes absetzen und sedimentieren lässt,
- das Eiweißsediment gefiltert wird,
- aus dem Filtrat durch die Einwirkung von Salzen, mit Vorteil Ammoniumsulfat, das Edestin in Lösungsform isoliert wird
und weiter das Edestin mit Dialysehilfe oder mit Säulenchromatographie auf eine Reinheit die höher als 95 %, mit Vorteil 99,9 % ist, gereinigt wird.

## Revendications

1. Moyen de remplacement du plasma sanguin,
**caractérisé par le fait que**
il se compose d'une substance végétale protéinée, l'edestin.

2. Moyen de remplacement du plasma sanguin conformément à la revendication n° 1,
**caractérisée par le fait que**
il est le produit des graines de chanvre.

3. Moyen de remplacement du plasma sanguin conformément à la revendication n° 1 ou n° 2,
**caractérisée par le fait que**
il est la composante d'une solution contenant 5 à 40% de masse d'edestin, 0,5 à 7 % de masse de substances anorganiques, 0,5 à 7 % de masse de substances organiques, hormones et d'autres substances pour 100 % de masse de solution colloïdale.

4. Méthode de production du moyen de remplacement du plasma sanguin conformément à la revendication n° 1,
**caractérisée par le fait que**
les graines végétales sont soit pressées à froid soit broyées et extraites sous pression du gaz et l'huile végétale extraite ou pressée est séparée, les graines pressées ou extraites étant, pour le dégraissage et la décoloration, extraites par l'effet de solvants organiques, la masse dégraissée et décolorée filtrée, puis soumise, dans une solution aqueuse, à l'action de sels anorganiques à une température de 20 à 60° C. La solution est filtrée et l'edestin isolé à partir du filtrat.

5. Méthode de production du moyen de remplacement du plasma sanguin conformément à la revendication n° 4,
**caractérisée par le fait que**
le processus de pressurage à froid et de broyage et le processus d'extraction sous pression du gaz sont appliqués aux graines de chanvre.

6. Méthode de production du moyen de remplacement du plasma sanguin conformément aux revendications n° 4 ou 5,
**caractérisées par le fait que**
les graines végétales sont extraites sous la pression du gaz carbonique.

7. Méthode de production du moyen de remplacement du plasma sanguin conformément à une des revendications 4 à 6,
**caractérisées par le fait que**
- l'on ajoute, à la substance dégraissée et décolorée, une solution saturée de sels anorganiques de 20 à 60° C de température, avec une préférence pour une température de 20° C,
- le mélange obtenu est brassé, à une température entre 20 à 60° C, avec une préférence pour une température de 20° C, pendant 0,01 à 2,5 heures, avec une préférence pour 0,5 heure,
- ensuite, on ajoute 0,1 à 30 % de masse de sel anorganique,
- le mélange obtenu est brassé, à une température entre 20 à 60° C pendant 0,6 à 60 minutes, avec une préférence pour une durée de 30 minutes,
- il est filtré, par l'intermédiaire de filtres chimiques,
- le filtrat est rapidement refroidi à la température de -50 à + 15 °C pour séparer l'edestin par précipitation sous forme cristalline,
- les cristaux bruts d'edestin sont versés dans l'eau distillée et sont séparés par filtration sous pression,
- à partir du filtrat obtenu, on crée une solution aqueuse avec l'edestin
ou
- à partir de la substance dégraissée et décolorée, on crée, en ajoutant de l'eau, une solution aqueuse de 20 à 60° C de température, avec préférence pour 5° C,
- on ajoute du sel anorganique dans la solution aqueuse, de préférence du chlorure de sodium, de manière à obtenir une solution de 1 à 20%, avec une préférence pour une solution à 5 %,
- le mélange est fortement brassé pendant 1 à 60 minutes, avec une préférence pour 30 minutes,
- on le laisse reposer et sédimenter afin de séparer le sédiment protéiné,
- le sédiment protéiné est filtré,
- l'edestin, sous forme de solution est isolé dans le filtrat par l'effet des sels, avec une préférence pour le sulfate d'ammonium
puis l'edestin est purifié par dialyse ou par chromatographie sur colonne pour obtenir une pureté supérieure à 95 %, avec une préférence à 99,9 %.
